# EUROPÄISCHE PATENTSCHRIFT

(11) **EP 3 975 957 B1**
(45) Veröffentlichungstag und Bekanntmachung des Hinweises auf die Patenterteilung: **24.07.2024**
(21) Anmeldenummer: 20726369.0
(22) Anmeldetag: 13.05.2020
(51) Int. Cl.: A61F 13/15, A61F 13/496, A61F 13/84, B65H 23/04, B65H 23/188, B65H 39/14

(54) **VERFAHREN ZUM HERSTELLEN VON INKONTINENZARTIKELN**
METHOD FOR PRODUCING INCONTINENCE ARTICLES
PROCÉDÉ DE FABRICATION D'ARTICLES D'INCONTINENCE

(30) Priorität: 27.05.2019 DE 102019114136
(43) Veröffentlichungstag der Anmeldung: 06.04.2022
(73) Patentinhaber: Paul Hartmann AG, 89522 Heidenheim (DE)
(72) Erfinder: BUCH, Tamara, 89077 Ulm (DE); EILERS, Jörg, 89179 Beimerstetten (DE); SCHMIDT, Ann-Cathrin, 06667 Weißenfels (DE); BEYRLE, Andreas, 89564 Nattheim (DE)
(74) Vertreter: DREISS Patentanwälte PartG mbB
(86) Internationale Anmeldenummer: PCT/EP2020/063366
(87) Internationale Veröffentlichungsnummer: WO 2020/239447

(56) Entgegenhaltungen:
- DE-A1- 102005 018 547
- US-A1- 2017 128 274
- US-A1- 2019 060 135

## Beschreibung

Die Erfindung betrifft ein Verfahren zum Herstellen von Inkontinenzartikeln für die Aufnahme von Körperausscheidungen mit einer Artikellängsrichtung und mit einer Artikelquerrichtung in einer Herstellungsmaschine, mit einem vorderen Bauchabschnitt und einem hinteren Rückenabschnitt, die in der Artikellängsrichtung zunächst voneinander beabstandet sind und dann zur Bildung eines in Quer- oder Hüftumfangsrichtung durchgehenden Bauch- und Rückenbands mit einer in Hüftumfangsrichtung geschlossenen Hüftöffnung aufeinandergefaltet und an beidseitigen Seitennahtbereichen herstellerseitig miteinander verbunden werden, und mit einem einen Absorptionskörper aufweisenden Schrittabschnitt, der sich in der Artikellängsrichtung zwischen Bauchabschnitt und Rückenabschnitt erstreckt, wobei der Schrittabschnitt, der Bauchabschnitt und der Rückenabschnitt gemeinsam jeweilige Beinöffnungen des Inkontinenzartikels begrenzen, wobei eine hintere Rückenabschnittskomponenten bildende erste Flachmaterialbahn und eine vordere Bauchabschnittskomponenten bildende zweite Flachmaterialbahn in einer Maschinenlängsrichtung, welche die spätere Artikelquerrichtung bildet, endlos zugeführt werden und wobei in einer Positionierstation für die Schrittabschnitte die jeweiligen Schrittabschnitte mit der späteren Artikellängsrichtung quer zu der Maschinenlängsrichtung angeordnet werden und mit der ersten und mit der zweiten Flachmaterialbahn für den bestimmungsgemäßen Gebrauch des Artikels verbunden werden, und wobei an dem Bauchabschnitt und/oder an dem Rückenabschnitt ein Kennzeichnungsmittel angebracht wird, wobei die erste Flachmaterialbahn und/oder die zweite Flachmaterialbahn in Maschinenlängsrichtung aufeinanderfolgende Orientierungsmarken aufweist. US 2017/128274 A1 und US 2019/060135 A1 betreffen ein Verfahren dieser Art.

Bei der Herstellung von in einer Herstellungsmaschine aus endlos zugeführten Flachmaterialbahnen hergestellten Artikeln war es schon bekannt, auf die Flachmaterialbahn aufgebrachte Orientierungsmarken ausgeführt als Druckmarken zu nutzen. So offenbart DE 10 2005 018 547 A1 die Verwendung von Druckmarken zum Verbinden eines Endes einer zunehmend aufgebrauchten Flachmaterialrolle mit der Flachmaterialbahn einer nachfolgenden neuen Rolle. Bei dem hier in Rede stehenden Verfahren zum Herstellen von Inkontinenzartikeln der eingangs genannten Art besteht u.a. die Anforderung, das erwähnte Kennzeichnungsmittel je herzustellendem Inkontinenzartikel an eine Flachmaterialbahn anzubringen und ferner den erwähnten Schrittabschnitt an vorbestimmter Stelle des herzustellenden Artikels zu positionieren und in dieser Position zu fügen. Der vorliegenden Erfindung liegt die Aufgabe zugrunde, das eingangs genannte Verfahren hinsichtlich seiner prozessgenauen Ausführbarkeit und hinsichtlich eines möglichst ausschussfreien oder ausschussarmen Betriebs zu verbessern.

Diese Aufgabe wird bei einem Verfahren der genannten Art erfindungsgemäß dadurch gelöst, dass in der Herstellungsmaschine eine jeweilige Orientierungsmarke mittels eines ersten Sensors erfasst wird und dass in Abhängigkeit vom Zeitpunkt einer jeweiligen Erfassung eine in der Maschinenlängsrichtung nachfolgende Druckvorrichtung zum Aufbringen des Kennzeichnungsmittels angesteuert wird, so dass das Kennzeichnungsmittel in der Maschinenlängsrichtung in vorbestimmter Relation zu der jeweiligen mittels des ersten Sensors erfassten Orientierungsmarke aufgebracht wird, und dass in der Herstellungsmaschine eine jeweilige Orientierungsmarke mittels eines zweiten Sensors erfasst wird und dass in Abhängigkeit vom Zeitpunkt einer jeweiligen Erfassung die erste und/oder die zweite Flachmaterialbahn beschleunigt oder verzögert oder mit gleichbleibender Geschwindigkeit weitertransportiert wird, derart dass die Anordnung der jeweiligen Schrittabschnitte in der Positionierstation in der Maschinenlängsrichtung in vorbestimmter Relation zu der jeweiligen mittels des zweiten Sensors erfassten Orientierungsmarke ausgeführt wird, und wobei mittels des ersten Sensors und mittels des zweiten Sensors dieselben Orientierungsmarken erfasst werden.

Nach der Erfindung werden bei aufgerollten Flachmaterialbahnen vorgesehene Orientierungsmarken durch Erfassung mittels des ersten Sensors genutzt, um das Kennzeichnungsmittel weitestgehend positionsgenau an vorbestimmter Stelle in Bezug auf den herzustellenden Inkontinenzartikel aufzudrucken und weiter durch Erfassen von Orientierungsmarken mittels des zweiten Sensors den absorbierenden Schrittabschnitt an ebenfalls weitestgehend genauer Position in Bezug auf den herzustellenden Inkontinenzartikel zu positionieren und in dieser Position dann für den bestimmungsgemäßen Gebrauch unlösbar zu fixieren. Auf diese Weise kann sowohl eine weitestgehend positionsgenaue Aufbringung des Kennzeichnungsmittels als auch eine weitestgehend positionsgenaue Anordnung und Fixierung des absorbierenden Schrittteils in prozessstabiler Weise erreicht werden.

Es erweist sich hierbei als vorteilhaft, wenn eine jeweilige Erfassung von Orientierungsmarken mittels des zweiten Sensors zeitlich nach einer jeweiligen Erfassung von Orientierungsmarken mittels des ersten Sensors ausgeführt wird. Dies impliziert dann in vorteilhafter Weise, dass zunächst das Kennzeichnungsmittel durch Ansteuerung der Druckvorrichtung in Abhängigkeit vom Zeitpunkt einer jeweiligen Erfassung der Orientierungsmarke positionsgenau aufgebracht wird und danach nach weiterer Erfassung einer jeweiligen Orientierungsmarke mittels des zweiten Sensors der Schrittteil in Abhängigkeit der jeweiligen Erfassung mittels des zweiten Sensors zeitlich nachfolgend, also an stromabwärts gelegener Position in Maschinenlängsrichtung positionsgenau aufgebracht und befestigt wird.

Der zweite Sensor ist hierfür vorzugsweise und zweckmäßigerweise in der Maschinenlängsrichtung der Druckvorrichtung nachfolgend angeordnet.

Die Erfassung der jeweiligen Orientierungsmarken mittels des ersten und des zweiten Sensors kann vom Grundsatz her in weitgehend beliebiger Weise erfolgen, indem die Orientierungsmarken eine durch den betreffenden Sensor detektierbare Eigenschaft aufweisen, welche der Sensor in ein Sensorsignal umsetzen bzw. ein Sensorsignal bei der Detektion der Orientierungsmarke aussenden kann, welches für weitergehende Steuerungszwecke eingesetzt werden kann. Es erweist sich diesbezüglich als vorteilhaft und einfach realisierbar, wenn diese Eigenschaft eine optisch wahrnehmbare Information umfasst, so dass der erste und/oder der zweite Sensor als optischer oder optoelektronischer Sensor ausgebildet werden können.

Für die aufeinanderfolgende, d.h. getaktete Ausführung von Verfahrensmaßnahmen im Zuge des Herstellungsverfahrens in der typischerweise schnelllaufenden Herstellungsmaschine erweist es sich als einfach und daher in vorteilhafter Weise realisierbar, wenn der erste Sensor mit einer elektronischen Steuervorrichtung, dabei bevorzugt mit einem Nockenschaltwerk einer elektronischen Steuervorrichtung, für die Druckvorrichtung zusammenwirkt, wobei ein weiterer Parameter von einer Transportgeschwindigkeit der Flachmaterialbahn in der Maschinenlängsrichtung gebildet wird. Die an sich bekannte Verwendung eines sensorgetriggerten Nockenschaltwerks gestattet im Zusammenwirken mit der elektronischen Steuervorrichtung eine zeitgenaue Ansteuerung der Druckvorrichtung für die zeitgenaue Ausführung des Druckprozesses zur Aufbringung des Kennzeichnungsmittels auf der an der Druckvorrichtung vorbeigeführten zu bedruckenden Flachmaterialbahn, wobei die Druckvorrichtung vorzugsweise ortsfest vorgesehen ist.

Hierbei erweist es sich als vorteilhaft, dass die Erfassung der jeweiligen Orientierungsmarke mittels des ersten Sensors einen Startzeitpunkt für die elektronische Steuervorrichtung, insbesondere für das Nockenschaltwerk einer elektronischen Steuervorrichtung, bildet und dass unter Verwendung dieses Startzeitpunkts ein Ereigniszeitpunkt für die Auslösung des Druckvorgangs durch die Druckvorrichtung ermittelt und der Druckvorgang zu diesem Ereigniszeitpunkt ausgelöst wird. Bei der Ermittlung des Steuerungszeitpunkts kann zur zeitgenauen Auslösung des Druckvorgangs in Bezug auf einen den jeweils herzustellenden Inkontinenzartikel bildenden Längsabschnitt der transportierten Flachmaterialbahn ein Zeitfenster vorgegeben werden, innerhalb dessen sich der Ereigniszeitpunkt für die Auslösung des Druckvorgangs befindet. Die Ausführung des Steuerungsvorgangs stellt dann sicher, dass sich der Ereigniszeitpunkt für die Auslösung des Druckvorgangs innerhalb eines vorbestimmten, vorzugsweise sehr engen Zeitfensters eines Takts, also in vorbestimmtem zeitlichem Abschnitt zu dem mittels des ersten Sensors bestimmten Startzeitpunkt befindet.

Mittels der Verwendung eines Nockenschaltwerks als Teil der elektronischen Steuervorrichtung kann in vorteilhafter Weise jedem Längsabschnitt der transportierten Flachmaterialbahn dem Grunde nach ein Ereigniszeitpunkt für die Auslösung des Druckvorgangs zugeordnet werden. Hierbei kann insbesondere auch unabhängig von der Erfassung der Orientierungsmarke des jeweiligen Längsabschnitts der Flachmaterialbahn, also beispielsweise falls der erste Sensor eine jeweilige Orientierungsmarke nicht erkennt, der Druckvorgang ausgelöst bzw. das Kennzeichnungsmittel innerhalb des Längsabschnitts aufgebracht werden, wenngleich der Positionierung des Kennzeichnungsmittels solchen Falls eine Ungenauigkeit anhaftet.

Dadurch, dass die Ansteuerung der Druckvorrichtung, also die Auslösung des Druckvorgangs, zeitlich gesteuert ausgehend von dem mittels des ersten Sensors erfassten Startzeitpunkt erfolgt, lässt sich bei bekannter Transportgeschwindigkeit der Flachmaterialbahn das Kennzeichnungsmittel an exakt intendierter vorgegebener Stelle aufbringen, d.h. in der Maschinenlängsrichtung in vorbestimmter Relation zu der Orientierungsmarke, d.h. in vorbestimmter räumlicher Anordnung zu der Orientierungsmarke in Bezug auf die Maschinenlängsrichtung, welche die Artikelquerrichtung des herzustellenden Artikels bildet. Die Positionierung oder Ausrichtung des Kennzeichnungsmittels quer zur Maschinenlängsrichtung, d.h. in Artikellängsrichtung relativ zu der Orientierungsmarke wird durch die typischerweise ortsfeste Anordnung des Druckwerks bzw. der Druckerdüsen in Bezug auf die vorbeigeführte Flachmaterialbahn realisiert. Diese Ausrichtung wird im einfachsten Fall beibehalten, jedenfalls wenn die Druckvorrichtung so ausgebildet ist, dass sie eine Erstreckung des Kennzeichnungsmittels quer zur Maschinenlängsrichtung bzw. Transportrichtung überfängt und abzudecken vermag. Dies schließt aber die Vorsehung komplexer querverschieblicher Druckwerke nicht aus.

Die vorbeschriebene Ansteuerung der Druckvorrichtung und damit die Aufbringung des Kennzeichnungsmittels erfolgt daher in Bezug auf jeden einzelnen herzustellenden Inkontinenzartikel nicht nur im Sinne eines Steuervorgangs, sondern vorzugsweise im Sinne eines Regelungsvorgangs, indem für jeden einzelnen Artikel sichergestellt wird, dass das Kennzeichnungsmittel innerhalb vorgegebener Grenzen aufgebracht wird, insbesondere wenn ein vorerwähntes Zeitfenster für den Ereigniszeitpunkt für die Auslösung des Druckvorgangs definiert ist. Es wird hier also nicht einer festgestellten zunehmenden oder abnehmenden Tendenz über viele Taktzyklen entgegengewirkt, sondern durch die Ausführung des beanspruchten Verfahrens ist es möglich, dass bei jedem einzelnen Inkontinenzartikel das Kennzeichnungsmittel in der vorbestimmten Relation der Orientierungsmarke aufgebracht wird.

Die Positionierung der absorbierenden Schrittteile wird, wie eingangs schon erwähnt, dadurch ausgeführt, dass eine jeweilige Orientierungsmarke mittels eines zweiten Sensors erfasst wird und dass in Abhängigkeit vom Zeitpunkt einer jeweiligen Erfassung die erste und/oder die zweite Flachmaterialbahn beschleunigt oder verzögert oder mit gleichbleibender Geschwindigkeit weitertransportiert wird derart, dass der betreffende Längsabschnitt der Flachmaterialbahn, welcher dem in diesem Takt herzustellenden Inkontinenzartikel entspricht, zu dem weitestgehend genau intendierten Zeitpunkt die Positionierstation erreicht, wo der betreffende Schrittabschnitt für diesen Inkontinenzartikel aufgebracht wird. Die Positionierstation ist hierbei typischerweise und vorzugsweise ortsfest vorgesehen. Es ist vorteilhaft möglich, dass dieselben Orientierungsmarken, die für die vorbeschriebenen Steuerungszwecke zum Aufbringen des Kennzeichnungsmittels verwendet wurden, auch für die weitestgehend genaue Anordnung der jeweiligen Schrittabschnitte an der Flachmaterialbahn verwendet werden können. Es wird mithin gewissermaßen ein und dieselbe Ausgangsgröße, nämlich die jeweiligen Orientierungsmarken bzw. deren Erfassung durch den ersten und zweiten Sensor verwendet, was die Genauigkeit der Anbringung des Kennzeichnungsmittels und der Anordnung des Schrittabschnitts unterstützt und Folgeungenauigkeiten ausschließt.

Es wurde festgestellt, dass beim Abrollen von endlos zugeführten Flachmaterialbahnen von einer oder mehreren Flachmaterialrollen auch bei zum Beispiel zuliefererseitig exakt äquidistant zueinander in der Bahnlängserstreckung aufgebrachten Orientierungsmarken der Orientierungsmarkenabstand in der Maschinenlängsrichtung beim Abrollen von der betreffenden Flachmaterialrolle variieren kann. Ohne die Ursache hierfür für alle Fälle und Gegebenheiten erklären zu wollen, wird anmelderseitig davon ausgegangen, dass dies auf die Lagerung der Flachmaterialrollen und auf kinetische Vorgänge und Spannungs- bzw. Zugeinwirkung im Zuge des Abrollens und Zuführens der Flachmaterialbahn in der Herstellungsmaschine zurückzuführen ist. Es wurde nämlich festgestellt, dass ausgehend von einer noch gefüllten neu angebrochenen Rolle beim Abrollen der Flachmaterialbahn eine Tendenz von anfänglich eher zu großem Orientierungsmarkenabstand in Maschinenlängsrichtung zu abnehmendem und schließlich eher zu kleinem Orientierungsmarkenabstand festzustellen ist. Im Zuge der Ausführung des erfindungsgemäßen Verfahrens erweist es sich daher als vorteilhaft, dass durch Erfassen der jeweiligen Orientierungsmarke mittels des zweiten Sensors und der anschließenden Geschwindigkeitsänderung der Flachmaterialbahn eine Veränderung von Orientierungsmarkenabständen ausgeglichen wird. Bei Bestehen einer Tendenz beispielsweise von eher zu großen hin zu eher zu kleinen Orientierungsmarkenabständen beim kontinuierlichen Abrollen der Flachmaterialbahnen von ihren Rollen oder Haspeln kann durch die erwähnten Steuerungsmaßnahmen, nämlich die gesteuerte Veränderung, d.h. Anpassung der Bahngeschwindigkeit der Flachmaterialbahnen einer solchen Tendenz in wirksamer Weise entgegengewirkt werden, und zwar ohne dass für jeden einzelnen Positioniervorgang in einem jeweiligen Takt eine exakt ausgeregelte Zielführung für die Zuführung der betreffenden Bahn in die Positionierstation ausgeführt werden muss. Hierdurch lässt sich der steuerungs- bzw. regeltechnische Aufwand reduzieren.

Das im Zuge der Herstellung mittels der Druckvorrichtung aufgebrachte Kennzeichnungsmittel kann eine an sich beliebige visuell wahrnehmbare Information im weitesten Sinn verkörpern oder vermitteln, insbesondere eine dekorative Information oder einen Verwendungshinweis für den Endverbraucher, wobei solchenfalls das Kennzeichnungsmittel typischerweise bei jedem herzustellenden Hygieneartikel identisch ausgebildet werden dürfte, was aber nicht zwingend erforderlich ist. Alternativ hierzu kann es sich als vorteilhaft erweisen, wenn als Kennzeichnungsmittel eine chargenindividualisierende und damit herstellungsspezifische Information, insbesondere eine Lotnummer, aufgebracht wird. In diesem Fall ist das betreffende Kennzeichnungsmittel nicht für alle aufeinanderfolgend herzustellenden Inkontinenzartikel dieselbe, sondern sie variiert von Produkt zu Produkt oder sie variiert in vorgegebener Weise von einer Gruppe umfassend eine Mehrzahl von Artikeln zu einer nachfolgenden Gruppe einer weiteren Mehrzahl von Artikeln, um anhand der herstellungsspezifischen Information dann im Nachhinein ermitteln zu können, welcher Charge bzw. welchem Herstellungszeitpunkt oder welcher Herstellungsmaschine ein beispielsweise schadhaftes Produkt zuzuordnen ist.

Es kann sich auch als vorteilhaft erweisen, wenn im Zuge der Herstellung der Inkontinenzartikel sowohl Kennzeichnungsmittel zur Vermittlung von dekorativen Informationen oder von Handlungshinweisen einerseits vorgesehen werden und chargenindividualisierende herstellungsspezifische Informationen andererseits in der erfindungsgemäßen Weise aufgebracht werden. Dies erleichtert nämlich die Auffindbarkeit der letzteren herstellungsspezifischen Informationen, die typischerweise nicht derart prominent ausgebildet werden, dass sie auf den ersten Blick visuell wahrgenommen werden. Vielmehr stellt sich bei hier in Rede stehenden Artikeln das Problem eines Zielkonflikts bei der Aufbringung von herstellungsspezifischen Informationen, deren visuelle Wahrnehmbarkeit nur in Einzelfällen eine Rolle spielt, in diesen Einzelfällen aber gleichwohl mit vertretbarem Aufwand wahrnehmbar sein sollen. Dadurch, dass eine derartige herstellungsspezifische Information als Kennzeichnungsmittel in der Maschinenlängsrichtung in vorbestimmter Relation zu einer Orientierungsmarke aufgebracht ist, ist deren visuelle Auffindbarkeit und Wahrnehmbarkeit verbessert, da Orientierungsmarken typischerweise so ausgebildet sind, dass sie ohne weiteres wahrgenommen werden können. Hierdurch kann auch eine Gewöhnung der beteiligten Verkehrskreise eher beigefügt werden, eine chargenindividualisierende Information in der Nähe einer leicht wahrnehmbaren Orientierungsmarke zu suchen.

Es kann sich auch als vorteilhaft erweisen, wenn die erste und/oder die zweite Flachmaterialbahn außer den aufgebrachten Orientierungsmarken je herzustellendem Inkontinenzartikel ein weiteres, insbesondere visuell wahrnehmbares Kennzeichnungsmittel aufweist, welches in vorbestimmter räumlicher Anordnung zu der Orientierungsmarke angeordnet ist. Dieses Kennzeichnungsmittel kann dann auch bereits zuliefererseitig zusammen mit den Orientierungsmarken auf die Flachmaterialbahn aufgebracht sein. In der Folge ist dann das im Zuge der Ausführung des erfindungsgemäßen Verfahrens innerhalb der Herstellungsmaschine aufgedruckte Kennzeichnungsmittel ebenfalls in vorbestimmter Relation oder räumlicher Anordnung nicht nur zu der Orientierungsmarke, sondern zu dem weiteren bereits aufgebrachten Kennzeichnungsmittel angeordnet. Wie zuvor bereits erwähnt, wäre es aber auch denkbar, dass mittels der Druckvorrichtung mehrere Kennzeichnungsmittel in vorbestimmter Relation, d.h. räumlicher Anordnung, zu der Orientierungsmarke aufgedruckt werden.

Die Herstellungsmaschine wird in vorteilhafter Weise mit einer Geschwindigkeit der ersten und der zweiten Flachmaterialbahnen von 100 bis 800 m/min, insbesondere 200-600 m/min betrieben.

Bei derart schnelllaufenden Herstellungsmaschinen ist es wünschenswert, dass im laufenden Betrieb eine zunehmend aufgebrauchte Flachmaterialrolle gegen eine neue ausgetauscht werden kann. Die eingangs erwähnte DE 10 2005 018 547 A1 beschreibt einen solchen Rollentausch im laufenden Betrieb.

Bei einer bevorzugten Ausführungsform des erfindungsgemäßen Verfahrens wird ein derartiger automatischer Rollenwechsel ebenfalls unter Verwendung von Orientierungsmarken, und zwar insbesondere und vorzugsweise unter Verwendung derselben Orientierungsmarken, die von dem ersten und/oder zweiten Sensor erfasst werden, unterstützt ausgeführt. Es wird hierfür vorgeschlagen, dass im laufenden Betrieb in einer Spleißstation der Herstellungsmaschine eine jeweilige die erste und/oder die zweite Flachmaterialbahn durch Abrollen zuführende Flachmaterialrolle gegen eine neue Flachmaterialrolle ausgetauscht werden kann, wobei die Flachmaterialbahn der zunehmend aufgebrauchten Flachmaterialrolle mit der Flachmaterialbahn der neuen Flachmaterialrolle prozesstauglich verbunden wird. Hierfür wird mittels eines weiteren Sensors eine Orientierungsmarke bei der Flachmaterialbahn der zunehmend aufgebrauchten Flachmaterialrolle erfasst und daran anschließend ein Ende der Flachmaterialbahn der zunehmend aufgebrauchten Flachmaterialrolle in vorbestimmter Verzögerung zum Stillstand gebracht, so dass sich die Orientierungsmarke in einem vorbestimmten Abstand in der Maschinenlängsrichtung zu einer nachfolgenden Orientierungsmarke der vorpositionierten Flachmaterialbahn der neuen Flachmaterialrolle befindet und die Flachmaterialbahnen dann prozesstauglich verbunden werden, so dass die Orientierungsmarken der entstehenden Flachmaterialbahn denselben vorbestimmten Abstand zueinander haben wie die Orientierungsmarken der Flachmaterialbahn der zunehmend aufgebrauchten Flachmaterialrolle.

Ein solcher sogenannter "Zerospeedsplice", bei welchem die zunehmend aufgebrauchte Flachmaterialrolle in vorbestimmter Verzögerung zum Stillstand gebracht wird, so dass die zunehmend aufgebrauchte Flachmaterialbahn zum Zeitpunkt des Verbindens ebenso wie die neue Flachmaterialbahn quasi vollständig still steht, bietet den Vorteil, dass das Verbinden der beiden Flachmaterialbahnen mit hoher Genauigkeit erfolgen kann. Als alternative Variante ist ein sogenannter "fliegender Splice" denkbar, wobei eine Bahngeschwindigkeit der zunehmend aufgebrauchten Flachmaterialbahn und eine Bahngeschwindigkeit der neuen Flachmaterialbahn aneinander angeglichen werden, so dass die zunehmend aufgebrauchte Flachmaterialbahn und die neue Flachmaterialbahn zum Zeitpunkt des Verbindens mit weitestgehend gleicher Geschwindigkeit in der Herstellungsmaschine geführt werden.

Es erweist sich als vorteilhaft, wenn der Spleißstation in der Maschinenlängsrichtung nachfolgend und dem ersten Sensor und der Druckstation vorgeordnet eine Bahnspeichereinrichtung vorgesehen und betrieben wird.

Weiter erweist es sich als vorteilhaft, wenn der Druckstation in der Maschinenlängsrichtung nachgeordnet und dem zweiten Sensor und der Ablege- und Fügevorrichtung vorgeordnet eine Bahnantriebseinrichtung vorgesehen und betrieben wird.

Mittels des weiteren Sensors werden also vorzugsweise dieselben Orientierungsmarken erfasst wie mit dem ersten und/oder zweiten Sensor.

Die Orientierungsmarke eines jeweiligen Längsabschnitts der Bauch- und/oder Rückenabschnittskomponenten bildenden Flachmaterialbahn, welcher dem in einem Takt herzustellenden Inkontinenzartikel entspricht, ist vorzugsweise zwischen einer vorgesehenen Oberkante des Kennzeichnungsmittels, und einer Hüftöffnungskante des herzustellenden Inkontinenzartikels angeordnet. Besonders bevorzugt ist dabei, dass die Orientierungsmarke zentriert zur Längsmittelachse des Inkontinenzartikels angeordnet ist. Eine andere Anordnung der Orientierungsmarke ist jedoch ebenso denkbar.

Weiter ist es besonders bevorzugt, wenn die Orientierungsmarke eine Farbe aufweist und/oder mit einem die Farbe bildenden Mittel gestaltet ist. Falls das Bauch- und/oder Rückenabschnittskomponenten bildende Flachmaterial ein weiteres bereits aufgebrachtes Kennzeichnungsmittel aufweist, ist es besonders bevorzugt, wenn die Orientierungsmarke eine dem weiteren bereits aufgebrachten Kennzeichnungsmittel identische Farbe aufweist und/oder mit einem identischen die Farbe bildenden Mittel gestaltet ist. Alternativ können jedoch auch unterschiedliche Mittel und/oder Verfahren, insbesondere unterschiedliche Beschichtungsarten oder -methoden, sowie unterschiedliche Farben der Beschichtung oder Arten der Beschichtung, z.B. zum einen eine Kleberbeschichtung und zum anderen ein Farbaufdruck, vorgesehen sein.

Insbesondere kann vorgesehen sein, dass die Orientierungsmarke durch eine flächenhaft erstreckte farbige Zone gebildet ist.

Der verwendete Begriff "farbig" bzw. "Farbe" bezieht sich auf die visuelle Wahrnehmungseigenschaft von aus dem Lichtspektrum abgeleiteten und als solche vom Menschen über das Auge erkennbare Lichtleistung. Farben sind typischerweise bekannt wie u.a. Schwarz, Rot, Blau, Grün, Gelb und Mischungen davon.

Die farbige Zone hebt sich somit bevorzugt von der grundlegenden Einfärbung, also der bereits vor Aufbringung der Orientierungsmarke vorherrschenden Farbe von den Bauch- und/oder Rückenabschnittskomponenten bildenden Flachmaterialien ab.

Weiterhin wird ein Inkontinenzartikel vorgeschlagen, welcher hergestellt oder herstellbar ist nach einem Verfahren wie vorstehend beschrieben.

Die herzustellenden Inkontinenzartikel haben in Quer- oder Hüftumfangsrichtung des Bauch- und/oder Rückenabschnitts eine elastische oder elastifizierte Ausbildung. Es können also entweder in sich flächenelastische Materialien oder in sich nicht dehnbare Flachmaterialien Verwendung finden, wobei die letzteren dann typischerweise mit elastischen oder elastifizierenden Elementen, insbesondere Lycra^{®}-Fäden, verbunden werden. In bevorzugter Weise sind die zur Bildung des Bauch- und Rückenabschnitts in der Herstellungsmaschine zugeführten Flachmaterialbahnen von elastisch dehnbaren oder undehnbaren Vliesmaterialien gebildet, wobei auch Vlies/Film-Materialen als Laminate verwendet werden können.

Die Vliesmaterialien von Bauchabschnitt und/oder Rückenabschnitt und/oder Bauch- und/oder Rückenabschnittskomponenten des Inkontinenzartikels umfassen vorzugsweise Vliesstoffmaterialien, wie Spinnvliese, Kardenvliese oder Through Air bonded Kardenvliese. Besonders bevorzugt umfassen die Vliesmaterialien von Bauchabschnitt und/oder Rückenabschnitt ein Spinnvliesmaterial. Die für Bauchabschnitt und/oder Rückenabschnitt eingesetzten Vliesmaterialien, insbesondere eine jeweilige Vlieslage haben vorteilhafterweise ein Flächengewicht von 10 - 30 g/m², weiter vorzugsweise von 12 - 25 g/m², weiter vorzugsweise 12-20g/m², weiter vorzugsweise von 12 - 18 g/m².

Als elastische oder elastifizierende Elemente bzw. Elastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra^{®}- oder Spandex^{®}-Fäden eingesetzt. Die Elastifizierungsmittel haben vorzugsweise eine Stärke von 300-1500 dtex, insbesondere von 500-1200 dtex, weiter insbesondere von 500-900 dtex.

Die Elastifizierungsmittel werden vorzugsweise unter einer Vorspannung von 1,5-6,0, insbesondere von 2,5-5,0 an chassisbildenden Hüllmaterialien, insbesondere Vliesmaterialien, des Bauchabschnitts und/oder Rückenabschnitts (Stretch-bonding Verfahren) fixiert. Die Vorspannung ist definiert als Dehnungsgrad eines gedehnten Elastifizierungsmittels gegenüber dem ungedehnten/relaxierten Ausgangszustand des Elastifizierungsmittels im Zustand des Aufbringens und Fixierens des Elastifizierungsmittels in der Herstellungsmaschine. Der Dehnungsgrad errechnet sich also als Verhältnis der gedehnten Länge L' (= Ausgangslänge L + ΔL) zur Ausgangslänge L, also L'/L.

Vorzugsweise sind erste Elastifizierungsmittel als im Wesentlichen parallel zur Querrichtung des Inkontinenzartikels verlaufend eingebracht, vorzugsweise in einem Abstand von 3-10 mm, weiter vorzugsweise von 3-8 mm. Vorzugsweise können weiter zweite Elastifizierungsmittel, insbesondere in einem schrittseitigen und den Beinöffnungen zugewandten Bereich des Bauchabschnitts und/oder des Rückenabschnitts vorgesehen sein, die sich ausgehend von den beiden Seitennahtbereichen in Richtung auf eine Längsmittelachse des Inkontinenzartikels erstrecken und dabei bogenförmig sich auffächernd mit zunehmendem Abstand voneinander verlaufen.

Weitere Merkmale, Einzelheiten und Vorteile ergeben sich aus den beigefügten Patentansprüchen und der zeichnerischen Darstellung und nachfolgenden Beschreibung einer bevorzugten Ausführungsform des erfindungsgemäßen Verfahrens.

In der Zeichnung zeigt:
- Figur 1: eine schematische Darstellung von hier relevanten Verfahrensschritten im Zuge der Ausführung des erfindungsgemäßen Verfahrens zum Herstellen von Inkontinenzartikeln;
- Figur 2: eine den Verfahrensschritten nach Figur 1 nachfolgende schematische Darstellung weiterer Verfahrensschritte im Zuge der Ausführung des Herstellungsverfahrens;
- Figur 3: eine schematische Darstellung von Verfahrensmaßnahmen im Bereich einer Spleißstation zur Ausführung eines Rollenwechsels einer zunehmend aufgebrauchten Flachmaterialrolle gegen eine neue Flachmaterialrolle, wobei eine prozesstaugliche Verbindung der Flachmaterialbahnen ausgeführt wird;
- Figur 4: schematisch einen Inkontinenzartikel hergestellt nach erfindungsgemäßem Verfahren, in der Draufsicht, nachdem die Seitennahtbereiche geöffnet wurden und der Artikel flach ausgebreitet wurde;
- Figur 5: eine Darstellung eines Längsschnittes des Inkontinenzartikels nach Figur 4.

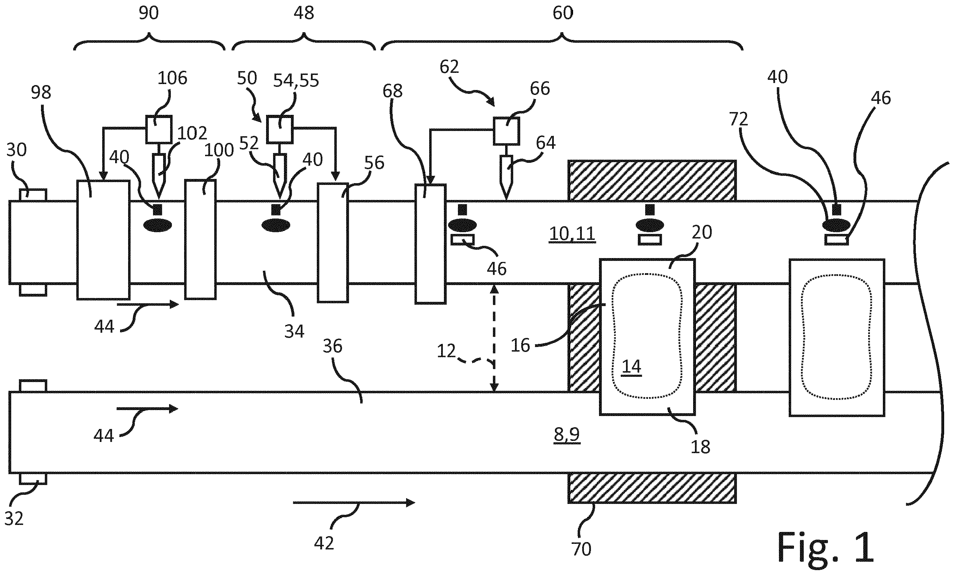

Die Figuren 1, 2 und 3 zeigen und verdeutlichen die Ausführung des erfindungsgemäßen Verfahrens zur Herstellung von Inkontinenzartikeln in einer schnelllaufenden Herstellungsmaschine, von der nur einzelne im Zusammenhang mit dem erfindungsgemäßen Verfahren stehende Komponenten und Maschinenbestandteile schematisch angedeutet sind.

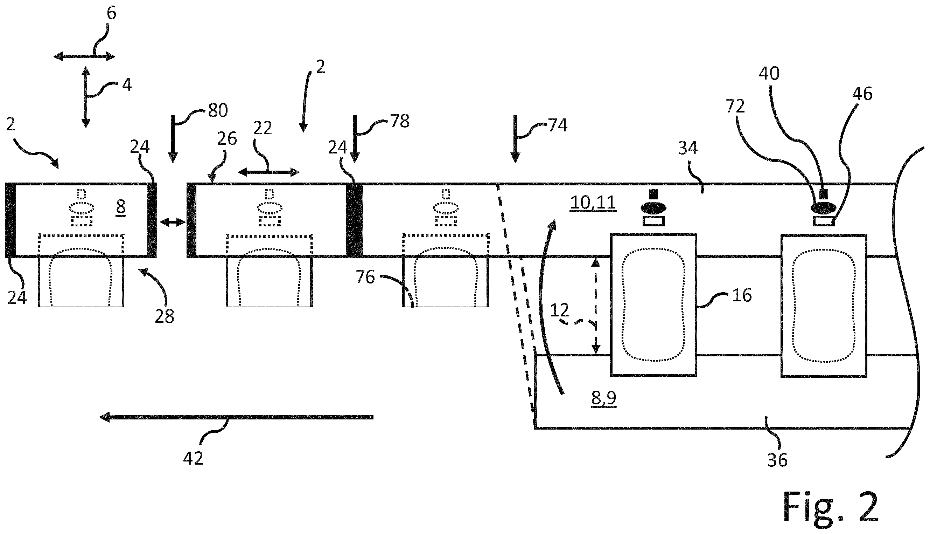

Die herzustellenden Inkontinenzartikel 2 haben eine Artikellängsrichtung 4 und eine Artikelquerrichtung 6 (Figur 2).

Sie haben ferner einen vorderen Bauchabschnitt 8 mit einer Bauchabschnittskomponente 9 und einen hinteren Rückenabschnitt 10 mit einer Rückenabschnittskomponente 11, die in diesem Ausführungsbeispiel während der Herstellung quer zur Maschinenlängsrichtung 42 voneinander beabstandet sind (Pfeil 12 in Figur 1). Im Zuge der Herstellung wird in diesem Ausführungsbeispiel ein einen Absorptionskörper 14 aufweisender Schrittabschnitt 16 auf die in der Längsrichtung 12 voneinander beabstandeten Bauchabschnitte 8 und Rückenabschnitte 10 bildende erste und zweite Flachmaterialbahnen 34, 36 in überlappender Anordnung aufgebracht und in einem vorderen und in einem hinteren Überlappungsbereich 18, 20 mit den den Bauchabschnitt 8 und den Rückenabschnitt 10 bildenden Flachmaterialbahnen 34, 36 für den bestimmungsgemäßen Gebrauch des Inkontinenzartikels insbesondere unlösbar verbunden. Danach (Figur 2) werden die die Bauchabschnitte 8 und die Rückenabschnitte 10 bildenden Flachmaterialbahnen 34, 36 aufeinandergefaltet und zur Bildung eines in Quer- oder Hüftumfangsrichtung 22 durchgehenden Bauch- und Rückenbands an beidseitigen Seitennahtbereichen 24 miteinander verbunden, so dass sie eine in Hüftumfangsrichtung 22 geschlossene Hüftöffnung 26 bilden und begrenzen und ein Inkontinenzartikel in Höschenform gebildet wird.

Bei dem Schrittabschnitt 16 handelt es sich um einen separaten in derselben Herstellungsmaschine oder einer zuführenden Herstellungsmaschine vorgefertigten absorbierenden Schrittteil, der typischerweise ein Topsheet, ein Backsheet und einen dazwischen angeordneten Absorptionskörper aufweist und zudem elastifizierende Elemente und sich zum Körper hin emporhebende seitliche als Seitenauslaufsperre dienende Komponenten sowie schrittelastifizierende Elemente aufweisen kann. Der Bauchabschnitt 8 und der Rückenabschnitt 10 sind in Quer- oder Hüftumfangsrichtung 22, d.h. in Artikelquerrichtung 6, elastisch dehnbar ausgebildet. Dies kann durch Einbringen von an sich bekannten streifen- oder fadenförmigen Elastifizierungsmitteln oder durch Verwendung von flächenelastischen Materialien erreicht werden. Im fertigen Zustand des Inkontinenzartikels (in Figur 2 links) begrenzt der Schrittabschnitt 16 zusammen mit dem Bauchabschnitt 8 und Rückenabschnitt 10 Beinöffnungen 28 des Artikels. Es versteht sich, dass der Inkontinenzartikel 2 weitere Funktionskomponenten und -elemente typischer absorbierender Inkontinenzartikel in Höschenform aufweisen kann.

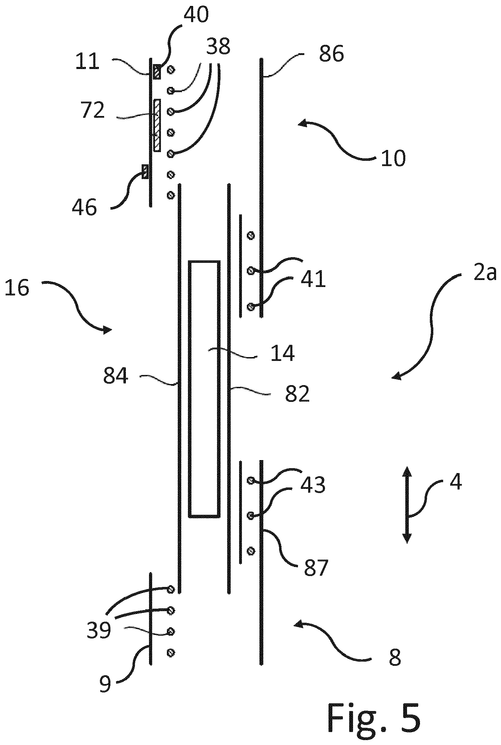

Zur Bildung einer Bauchabschnittskomponente 9 des späteren Bauchabschnitts 8 und einer Rückenabschnittskomponente 11 des späteren Rückenabschnitts 10 wird beispielhaft jeweils eine Flachmaterialrolle 30, 32 mit einer darauf aufgewickelten endlosen Flachmaterialbahn 34, 36, beispielhaft und vorzugsweise in Form einer Vliesstoffbahn oder Vliesstoffverbundbahn, abrollbar vorgesehen. Es wäre auch denkbar, dass eine einzige Flachmaterialrolle vorgesehen ist, die zur Bildung der jeweiligen Bauch- und Rückenabschnitte 8, 10 in Teilbahnen getrennt wird. Denkbar und vorteilhaft können der Rückenabschnitt 10 und der Bauchabschnitt 8 jeweils auch von mehr als einer Rückenabschnittskomponente 11 bzw. Bauchabschnittskomponente 9 gebildet sein, wie weiter unten mit Bezug zu Figur 5 näher erläutert werden wird.

Im dargestellten Fall ist die auf der Flachmaterialrolle 30 aufgewickelte Flachmaterialbahn 34 mit Orientierungsmarken 40 versehen, die in einer Maschinenlängsrichtung 42 aufeinanderfolgend und mit wohldefiniertem vorgegebenem Abstand in der Maschinenlängsrichtung 42 voneinander auf der Flachmaterialbahn 34 aufgebracht sind. Die Maschinenlängsrichtung 42 entspricht dabei einer Bahnlängsrichtung 44 der endlosen Flachmaterialbahnen 34, 36, wenn diese abgerollt und in der Herstellungsmaschine kontinuierlich transportiert werden.

Diese Orientierungsmarken 40 werden im Zuge der Ausführung des erfindungsgemäßen Verfahrens sensortechnisch erfasst und zur positionsgenauen Anbringung eines Kennzeichnungsmittels 46 sowie zur positionsgenauen Anordnung eines jeweiligen Schrittabschnitts 16 auf der ersten und zweiten Flachmaterialbahn 34, 36 verwendet, was nachfolgend beschrieben wird:
Die Aufbringung des Kennzeichnungsmittels 46 im beispielhaft dargestellten Fall im Rückenabschnitt 10 des Inkontinenzartikels erfolgt in einer Druckstation 48 der Herstellungsmaschine. Die Druckstation 48 umfasst eine erste Sensorvorrichtung 50 mit einem Sensor 52 zum optischen Erfassen der vorbeitransportierten Orientierungsmarke 40, eine mit dem ersten Sensor 50 zusammenwirkende elektronische Steuervorrichtung 54 und eine Druckvorrichtung 56 zum Aufdrucken des Kennzeichnungsmittels 46 auf die Flachmaterialbahn 34.

Mittels des ersten Sensors 52 wird eine jeweilige auf der vorbeitransportierten ersten Flachmaterialbahn 34 aufgebrachte Orientierungsmarke 40 optisch erfasst. Es wird hierbei der Zeitpunkt einer jeweiligen Erfassung detektiert und durch die elektronische Steuervorrichtung 54 als Startzeitpunkt für die Ermittlung eines Ereigniszeitpunkts für die Auslösung des Druckvorgangs durch die Druckvorrichtung 56 verarbeitet, wobei die Bahngeschwindigkeit als weiterer Steuerparameter verwendet wird. Es besteht die Aufgabe, dass das Kennzeichnungsmittel 46 in der Maschinenlängsrichtung 42 in vorbestimmter Relation zu der jeweiligen Orientierungsmarke 40 aufgedruckt wird. Hierbei kann es erwünscht sein, dass das Kennzeichnungsmittel 46 wie in den Figuren dargestellt exakt unterhalb der Orientierungsmarke 40 aufgebracht wird oder hierzu in der Maschinenlängsrichtung 44 vorgeordnet oder nachgeordnet aufgebracht wird. Die Anordnung quer zur Maschinenlängsrichtung 44 erfolgt vorzugsweise durch prozessfeste Ausrichtung und Anordnung des Druckwerks bzw. von Strahldüsen der Druckvorrichtung 56 in Bezug auf die vorbeitransportierte Flachmaterialbahn 34. In Abhängigkeit vom Zeitpunkt einer jeweiligen Erfassung einer vorbeitransportierten Orientierungsmarke 40 durch den ersten Sensor 52 und unter Berücksichtigung der bekannten Bahngeschwindigkeit der ersten Flachmaterialbahn 34 wird mittels der elektronischen Steuervorrichtung 54 ermittelt, wann der Bereich der Flachmaterialbahn 34, auf dem das Kennzeichnungsmittel 46 positionsgenau aufgedruckt werden soll, die Druckvorrichtung 56 erreicht. Dieser Zeitpunkt wird als Ereigniszeitpunkt für die Auslösung des Druckvorgangs verwendet, und mittels der elektronischen Steuervorrichtung 54 wird die Druckvorrichtung 56 dann derart angesteuert, dass der Druckvorgang zu diesem Ereigniszeitpunkt ausgelöst wird. Auf diese Weise lässt sich eine positionsgenaue Aufbringung des Kennzeichnungsmittels 46 für jeden herzustellenden Inkontinenzartikel ausführen, wobei auch variierende Orientierungsmarkenabstände nicht zu Ungenauigkeiten bei der Aufbringung des Kennzeichnungsmittels 46 führen.

Nach einer bevorzugten Ausführungsform umfasst die elektronische Steuervorrichtung 54 ein sogenanntes Nockenschaltwerk 55, bei dem durch Erfassen einer Orientierungsmarke 40 der schon erwähnte Startzeitpunkt für den anstehenden Takt gesetzt wird. Ausgehend von diesem Startzeitpunkt wird durch das Nockenschaltwerk 55 der erwähnte Ereigniszeitpunkt für die Auslösung des Druckvorgangs durch die Druckvorrichtung 56 ermittelt und ein Steuerbefehl an die Druckvorrichtung 56 gegeben. Die Verwendung eines Nockenschaltwerks hat den Vorteil, dass sich von Taktzyklus zu Taktzyklus keine Ungenauigkeiten aufaddieren, da jeder Zyklus durch die Erfassung einer Orientierungsmarke 40 als Startzeitpunkt für die Auslösung von nachfolgenden Steuerungsmaßnahmen neu begonnen wird. Die Verwendung eines Nockenschaltwerks ermöglicht außerdem, den Druckvorgang auch dann auszulösen, wenn innerhalb eines Taktes durch den ersten Sensor keine Orientierungsmarke erkannt wird, wenngleich der Positionierung des Kennzeichnungsmittels solchen Falls eine Ungenauigkeit anhaftet.

Die Anordnung eines jeweiligen Schrittabschnitts 16 an der ersten und zweiten Flachmaterialbahn 34, 36 wird erfindungsgemäß ebenfalls sensorunterstützt in einer Positionierstation 60 ausgeführt. Hierdurch kann einer von der Anmelderin festgestellten Tendenz von beim Abrollen der Flachmaterialbahnen sich einstellenden Veränderungen der Abstände der Orientierungsmarken 40 in Maschinenlängsrichtung 42 voneinander entgegengewirkt werden. Es wurde festgestellt, dass sich die Orientierungsmarkenabstände zu Beginn einer von einer Flachmaterialrolle 30, 32 abgezogenen Flachmaterialbahn 34, 36 von eher zu großen Orientierungsmarkenabständen hin zu eher zu geringen Orientierungsmarkenabständen kontinuierlich verändern, was auf Lagerprozesse und kinematische Einflüsse im Zuge des Abziehens und Zuführens der Flachmaterialbahnen zurückgeführt wird. Auch eine bei der Herstellung oder Verarbeitung der Flachmaterialbahn 34, 36 hervorgerufene kontinuierliche Vergrößerung der Orientierungsmarkenabstände ist denkbar und kann mittels des dargestellten Verfahrens ausgeglichen werden. Die in Figur 1 schematisch dargestellte Positionierstation 60 umfasst eine zweite Sensorvorrichtung 62 mit einem zweiten Sensor 64 und einer elektronischen Steuervorrichtung 66 und eine durch die elektronische Steuervorrichtung 66 gesteuerte Bahnantriebsvorrichtung 68, mittels derer die Flachmaterialbahn 34 beschleunigt oder verzögert oder mit gleichbleibender Geschwindigkeit weitertransportiert werden kann. Diese Bahnantriebsvorrichtung 68 ist nur schematisch an der Stelle des Bezugszeichens 68 angedeutet. Sie umfasst weitere Antriebskomponenten, insbesondere weitere Rollen, die in vorzugsweise schlupffreier Transportverbindung mit der Flachmaterialbahn 34 stehen.

Mittels des zweiten Sensors 64 wird eine jeweilige auf der vorbeitransportierten Flachmaterialbahn 34 angebrachte Orientierungsmarke 40 vorzugsweise optisch erfasst, und ein Zeitpunkt der Erfassung wird in der Steuervorrichtung 66 verarbeitet und für die Ansteuerung der Bahnantriebsvorrichtung 68 verwendet. Wiederum wird unter Berücksichtigung einer bekannten momentanen Bahngeschwindigkeit und eines maschinenfesten Abstands zwischen dem zweiten Sensor 64 und einem Referenzpunkt der Positionierstation 60, wo die jeweiligen Schrittabschnitte 16 an vorbestimmter Stelle in der Maschinenlängsrichtung 42 auf die Flachmaterialbahnen 34, 36 abgelegt werden, der Zeitpunkt ermittelt, zu dem unter Zugrundelegung der momentanen Bahngeschwindigkeit ein vorbestimmter Bereich der Flachmaterialbahn 34, auf dem ein betreffender Schrittabschnitt 16 abgelegt werden soll, die Ablege- und Fügevorrichtung 70 erreicht. In Abhängigkeit davon wird mittels der elektronischen Steuervorrichtung 62 ermittelt, ob die Flachmaterialbahn beschleunigt oder verzögert oder mit gleichbleibender Geschwindigkeit weitertransportiert werden muss, damit der vorbestimmte Bereich zeitgenau die Ablege- und Fügevorrichtung 70 erreicht, so dass der betreffende Schrittabschnitt 16 in der Maschinenlängsrichtung 42 positionsgenau, d.h. in vorbestimmter Relation zu der Orientierungsmarke 40, auf den Flachmaterialbahnen 34, 36 abgelegt und in den schon erwähnten Überlappungsbereichen 18, 20 mit den Flachmaterialbahnen 34, 36 unlösbar verbunden werden kann. Vorzugsweise ist der zweite Sensor 64 zwischen der Bahnantriebsvorrichtung 68 und der Ablege- und Fügevorrichtung 70 positioniert und dabei insbesondere und vorzugsweise nahe, weiter insbesondere direkt vorgeordnet der Ablege- und Fügevorrichtung 70. Das hat den Vorteil, dass die Kontrolle der Orientierungsmarkenabstände in räumlicher und zeitlicher Nähe zum Ablege- und Fügeereignis erfolgt, wodurch Ungenauigkeiten entgegengewirkt werden kann. Denkbar ist jedoch auch eine umgekehrte Anordnung des zweiten Sensors 64 und der Bahnantriebsvorrichtung 68.

Bei dem Kennzeichnungsmittel 46 handelt es sich insbesondere und vorzugsweise um eine chargenindividualisierende Information, wie z.B. eine Lotnummer. Durch diese chargenindividualisierende Information kann dann an einem späteren Zeitpunkt festgestellt und zurückverfolgt werden, wann und wo, also innerhalb welcher Produktionscharge, ein betreffender Inkontinenzartikel hergestellt wurde. Das Kennzeichnungsmittel stellt daher eine herstellungsspezifische Information über den Hygieneartikel und dessen Herstellung zur Verfügung. Da derartige Informationen typischerweise nur selten benötigt werden, ist es nicht erforderlich, das Kennzeichnungsmittel, welches diese Information verkörpert oder vermittelt, auf den ersten Blick prominent oder ins Auge springend vorzusehen. Dadurch, dass durch die erfindungsgemäße Herstellung das Kennzeichnungsmittel aber an exakt vorbestimmter Stelle in Relation zu einer typischerweise gut optisch erfassbaren Orientierungsmarke aufgebracht ist, vermag diese Orientierungsmarke auch die Auffindung des Kennzeichnungsmittels zu unterstützen. Dies erweist sich insbesondere beim Aufdruck des Kennzeichnungsmittels auf elastifizierte Materialien, insbesondere Vliesstoffbahnen, als vorteilhaft, welche sich infolge der Elastifizierung im fertigen Zustand zusammenziehen, also raffen oder kräuseln, was die Wahrnehmbarkeit des Kennzeichnungsmittels erschwert.

Es sei an dieser Stelle noch darauf hingewiesen, dass das Kennzeichnungsmittel nicht zwingend eine von Produktcharge zu Produktcharge variierende Information vermitteln muss, sondern auch eine für mehrere Chargen gleichbleibende, insbesondere dekorative Information oder einen an den Endverbraucher gerichteten Verwendungshinweis darstellen kann. Im beispielhaft dargestellten Fall handelt es sich bei dem Kennzeichnungsmittel 46 aber tatsächlich um eine chargenindividualisierende Information; jedoch ist ein weiteres insbesondere visuell wahrnehmbares Kennzeichnungsmittel 72 vorgesehen, welches im beispielhaften Fall wie die Orientierungsmarken 40 insbesondere zuliefererseitig auf die endlose Flachmaterialbahn 34, und zwar in vorbestimmter räumlicher Relation und Anordnung zu einer jeweiligen Orientierungsmarke 40 aufgebracht ist. In der Folge wird dann durch das erfindungsgemäße Verfahren überdies sichergestellt, dass das Kennzeichnungsmittel 46, welches die chargenindividualisierende Information bereitstellt, ebenfalls in vorbestimmter Relation und Anordnung zu dem weiteren Kennzeichnungsmittel 72 auf der Flachmaterialbahn 34 und damit im herzustellenden Rückenabschnitt 10 des Inkontinenzartikels vorgesehen ist. Somit kann auch das weitere Kennzeichnungsmittel 72 als "Auffindungshilfe" für die durch das chargenindividualisierende Kennzeichnungsmittel 46 vermittelte herstellungsspezifische Information dienen.

Im weiteren Verlauf der Herstellung in der schnelllaufenden Herstellungsmaschine folgt, gegebenenfalls nach dem Einbringen weiterer Artikelkomponenten, wie z.B. Elastifizierungsmittel, Kleber, Vlies- und/oder Folienabschnitten, eine Bahnfaltvorrichtung 74 (Figur 2), mittels derer die Flachmaterialbahnen 34, 36 um eine in Artikelquerrichtung 6 verlaufende Schrittmittelachse 76 auf sich selbst gefaltet werden, so dass die Bauchabschnitte 8 und Rückenabschnitte 10 der herzustellenden Artikel übereinander und aneinander anliegen. Mittels einer Seitennahtfügevorrichtung 78 werden dann die schon erwähnten Seitennahtbereiche 24 des Bauch- und Rückenabschnitts 8, 10 mittels dem Fachmann an sich bekannter Fügeverfahren, insbesondere durch Thermoschweißen, Ultraschallschweißen, Nähen, Siegeln oder Kleben miteinander verbunden. Im Anschluss daran oder möglicherweise auch gleichzeitig erfolgt mittels einer Trenn-/Vereinzelungsvorrichtung 80 die Vereinzelung der Artikel, und zwar in Artikellängsrichtung 4 durch einen jeweiligen Seitennahtbereich 24 hindurch. Der Artikel wird dann etwaigen weiteren Faltvorgängen zugeführt und typischerweise in vorgegebener Anzahl verpackt.

Im Rahmen der vorliegenden Erfindung werden Komponenten als miteinander verbunden definiert, wenn die Komponenten direkt oder indirekt aneinander kraft- oder form- oder stoffschlüssig gefügt sind. Indirekt sind eine erste und eine zweite Komponente dann aneinandergefügt, wenn eine erste Komponente nicht unmittelbar an eine zweite Komponente gefügt ist, sondern gleichsam mittelbar über mindestens eine weitere Komponente.

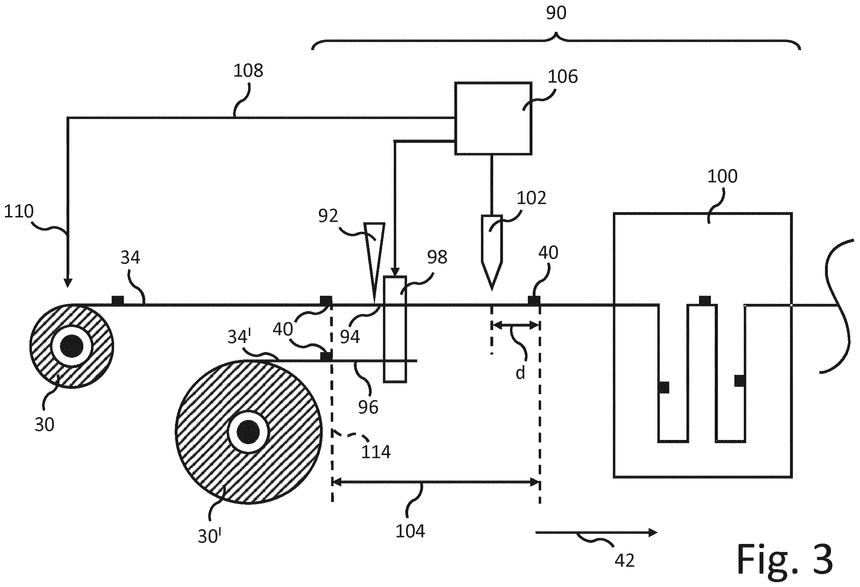

Es wird nun unter Bezugnahme auf Figur 3 ein Rollenwechsel einer zunehmend aufgebrauchten Flachmaterialrolle 30 gegen eine neue Flachmaterialrolle 30' im laufenden Betrieb der Herstellungsmaschine in der Ausführung als "Zerospeedsplice" erläutert. Figur 3 zeigt eine Seitenansicht auf einen in Figur 1 links dargestellten Bereich der Herstellungsmaschine, beginnend mit einer sich kontinuierlich abrollenden Flachmaterialrolle 30. Von dieser Flachmaterialrolle wird die Flachmaterialbahn 34 in Maschinenlängsrichtung 42 kontinuierlich abgerollt. Bevor sie zu der bereits im Zusammenhang mit Figur 1 beschriebenen Druckstation 48 geführt wird, durchläuft die Flachmaterialbahn 34 eine Spleißstation 90. In der Spleißstation 90 wird eine zunehmend aufgebrauchte Flachmaterialbahn 34 sensorunterstützt zum Stillstand gebracht und mittels eines Trennmessers 92 von der möglichst vollständig abgerollten Flachmaterialrolle 30 abgetrennt, so dass ein hinterer Endbereich 94 der ersten Flachmaterialbahn 34 gebildet wird. Währenddessen befinden sich eine neue Flachmaterialrolle 30' und ein hiervon abgerollter vorderer Endbereich 96 der Flachmaterialbahn 34' in Wartestellung, insbesondere und vorzugsweise nahe und parallel zu der Bahnebene der Flachmaterialbahn 34 bzw. des hinteren Endbereichs 94 der Flachmaterialbahn 34. Der hintere Endbereich 94 und der vordere Endbereich 96 werden dann in Anlage aneinander gebracht und mittels einer Spleiß- oder Fügevorrichtung 98 prozesstauglich mittels dem Fachmann an sich bekannter Fügeverfahren, insbesondere durch Thermoschweißen, Ultraschallschweißen, Nähen, Siegeln oder Kleben miteinander verbunden. Dies geschieht vorzugsweise im stillgesetzten Zustand der ersten Flachmaterialbahn 34. Währenddessen erfolgt der nicht unterbrochene Herstellungsprozess kontinuierlich weiter, indem bei der Spleißstation 90 eine Bahnspeichereinrichtung 100 mit mehreren zueinander bewegbaren Umlenkrollen in an sich bekannter Weise den Stillstand der Bahn während des Spleiß- oder Fügevorgangs ermöglicht, indem von dort die erforderliche Bahnlänge in der Maschinenlängsrichtung 42 ausgegeben wird.

Nach einer Ausführungsform des erfindungsgemäßen Verfahrens wird die vorstehend beschriebene Trennung der aufgebrauchten Flachmaterialbahn 34 und die Verbindung mit der neuen Flachmaterialbahn 34' durch Erfassung einer oder mehrerer Orientierungsmarken 40 mittels eines weiteren Sensors 102 gesteuert derart, dass bei den verbundenen Flachmaterialbahnen 34 und 34' ein gleichbleibender vorbestimmter Orientierungsmarkenabstand 104 realisiert wird, so dass das weitergehende Herstellungsverfahren quasi unbeeinflusst durch den Rollenwechsel fortgesetzt werden kann. Hierfür ist der erwähnte weitere Sensor 102 vorgesehen, der ein Signal an eine elektronische Auswerte- und Steuervorrichtung 106 gibt. Wenn ein Rollenwechsel ansteht und ausgeführt werden soll, so wird die Erfassung einer an dem Sensor 102 vorbeitransportierten Orientierungsmarke 40 von der elektronischen Steuervorrichtung 106 als Signal zum genau vorbestimmten Verzögern und Stillsetzen der Flachmaterialbahn 34 verarbeitet. Hierfür wird durch die elektronische Steuervorrichtung 106 ein Ansteuerbefehl 108 an eine Flachmaterialbahnantriebsvorrichtung 110 gegeben, die hier lediglich durch einen auf die Flachmaterialrolle 30 zeigenden Pfeil verdeutlicht ist, und die mehrere synchron miteinander angetriebene Mittel, wie insbesondere Antriebsrollen, umfasst, welche die Flachmaterialbahn 34 von der Rolle abziehen und weitertransportieren, jedoch in den Figuren nicht dargestellt sind. Bei Erfassen der Orientierungsmarke 40 mittels des Sensors 102 wird also der Ansteuerbefehl 108 zum Verzögern und Stillsetzen an die Flachmaterialbahnantriebsvorrichtung 110 gegeben mit der Folge, dass die Bahn in vorbestimmter Weise verzögert und stillgesetzt wird, derart, dass die Bahn und die soeben erfasste Orientierungsmarke 40 um eine genau vorgegebene Distanz d bis zum Stillstand weitertransportiert wird. Die erfasste Orientierungsmarke 40 hat dann einen Abstand d von dem Sensor 102. Der Sensor 102 sowie die Spleiß- oder Fügevorrichtung 98 haben jeweils einen bekannten vorgegebenen Abstand von der nachfolgenden Orientierungsmarke 40, da ja der Orientierungsmarkenabstand 104 bei der Flachmaterialbahn 34 bekannt ist. Die Anordnung der Komponenten und der vorgegebene Verzögerungsweg bis zum Stillstand, also die Distanz d, werden so vorgegeben, dass sich die nachfolgende Orientierungsmarke 40 in der Maschinenlängsrichtung 42 an derselben Stelle, jedoch noch parallel versetzt zu einer entsprechenden ersten Orientierungsmarke 40 des vorderen Endbereichs 96 der Flachmaterialbahn 34', befindet. Diese Warteposition der ersten Orientierungsmarke 40 der Flachmaterialbahn 34' ist in der Figur 3 durch eine gestrichelte Linie 114 orthogonal zur Maschinenlängsrichtung 42 angedeutet. Sobald sich die beiden Orientierungsmarken 40 an der durch die gestrichelte Linie 114 bezeichneten Warteposition befinden, werden der vordere Endbereich 96 und der hintere Endbereich 94 in Anlage aneinander gebracht und mittels der Spleiß- oder Fügevorrichtung 98 prozesstauglich miteinander verbunden. Die neue Flachmaterialrolle 30' wird im Zuge dessen an die Position der aufgebrauchten und mittlerweile entfernten Flachmaterialrolle 30 gebracht. Im Wesentlichen unmittelbar an den Fügevorgang anschließend wird mittels der elektronischen Steuervorrichtung 106 die Bahnantriebsvorrichtung 110 zur Aufnahme des normalen Förderbetriebs angesteuert.

Das in Figuren 1, 2 und 3 schematisch dargestellte Verfahren kann eine oder mehrere weitere Sensorvorrichtungen und/oder Steuervorrichtungen und/oder Regelungsvorrichtungen und/oder Verarbeitungsvorrichtungen aufweisen, beispielsweise können weitere Flachmaterialbahnen, die elastisch dehnbare oder undehnbare Vliesmaterialien umfassen, und/oder elastische oder elastifizierende Elemente, insbesondere Lycra^{®}-Fäden oder Vlies/Film-Materialien, insbesondere Laminate zugeführt und mit der dargestellten ersten und/oder zweiten Flachmaterialbahn 34, 36 und/oder dem Schrittabschnitt 14 insbesondere durch die vorgenannten dem Fachmann an sich bekannten Fügeverfahren verbunden werden.

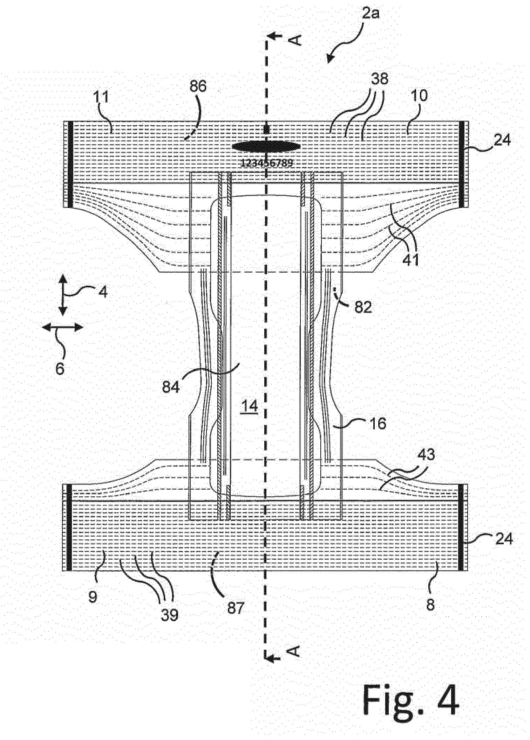

Figuren 4 und 5 zeigen schematisch einen weiteren Inkontinenzartikel 2a hergestellt oder herstellbar nach dem erfindungsgemäßen Verfahren, und zwar nachdem die Seitennahtbereiche 24 geöffnet wurden und der Artikel flach ausgebreitet wurde. Figur 5 ist hierbei eine schematische Schnittansicht längs durch den Inkontinenzartikel entlang der Linie A-A. Soweit dem Verständnis zuträglich sind den Elementen des Inkontinenzartikels 2a gleiche Bezugsziffern zugeordnet wie in den Figuren 1-3. Der Inkontinenzartikel 2a hat eine Artikellängsrichtung 4, eine Artikelquerrichtung 6, einen eine vordere Bauchabschnittskomponente 9 umfassenden vorderen Bauchabschnitt 8, einen eine hintere Rückenabschnittskomponente 11 umfassenden hinteren Rückenabschnitt 10 und einen Schrittabschnitt 16. Der Schrittabschnitt 16 umfasst ein flüssigkeitsundurchlässiges Backsheet 82, das insbesondere ein atmungsaktives, jedoch flüssigkeitsdichtes Folienmaterial umfasst, und ein flüssigkeitsdurchlässiges Topsheet 84, insbesondere aus einem Vliesstoffmaterial. Zwischen dem Backsheet 82 und dem Topsheet 84 ist ein Absorptionskörper 14 angeordnet. Im beispielhaft dargestellten Fall bilden das Backsheet 82 und das Topsheet 84 in Artikellängsrichtung 4 und Artikelquerrichtung 6 einen Überhang über den Absorptionskörper 14 und sind in den Bereichen des Überhangs direkt miteinander verbunden.

Der Rückenabschnitt 10 als auch der Bauchabschnitt 8 umfassen in dieser Variante des Inkontinenzartikel 2a neben der Rückenabschnittkomponente 11 und der Bauchabschnittkomponente 9 weitere Komponenten. Zur Elastifizierung eines hüftöffnungsnahen Bereichs des Rückenabschnitts 10 in Quer- oder Hüftumfangsrichtung 6 sind in diesem Beispiel erste elastische Fäden 38, vorzugsweise Lycra^{®}-Fäden, in vorgespanntem Zustand zwischen der Rückenabschnittskomponente 11 und einer die körperzugewandte Seite des Rückenabschnitts 10 bildenden Vliesmaterialkomponente 86 eingebracht und dort sowohl an die Rückenabschnittskomponente 11 als auch an die Vliesmaterialkomponente 86 gefügt. Zur Elastifizierung eines hüftöffnungsnahen Bereichs des Bauchabschnitts 8 in Quer- oder Hüftumfangsrichtung sind zweite elastische Fäden 39, vorzugsweise Lycra^{®}-Fäden, in vorgespanntem Zustand zwischen der Bauchabschnittskomponenten 9 und einer die körperzugewandte Seite des Bauchabschnitts 8 bildenden Vliesmaterialkomponente 87 eingebracht und dort sowohl an die Bauchabschnittskomponente 9 als auch an die Vliesmaterialkomponente 87 gefügt. Zur Elastifizierung eines hüftfernen Bereichs des Rückenabschnitts 10 in Quer- oder Hüftumfangsrichtung, dabei mit einer Komponente in der Längsrichtung 4, sind in diesem Beispiel dritte elastische Fäden 41, vorzugsweise Lycra^{®}-Fäden, in vorgespanntem Zustand an der die körperzugewandte Seite des Rückenabschnitts 10 bildenden Vliesmaterialkomponente 86 angefügt. Zur Elastifizierung eines hüftöffnungsfernen Bereichs des Bauchabschnitts 8 in Quer- oder Hüftumfangsrichtung, dabei mit einer Komponente in der Längsrichtung 4, sind vierte elastische Fäden 43, vorzugsweise Lycra^{®}-Fäden, in vorgespanntem Zustand an der die körperzugewandte Seite des Bauchabschnitts 8 bildenden Vliesmaterialkomponente 87 angefügt.

Der Schrittabschnitt 16 ist in dieser Ausführungsform mit dem Rückenabschnitt 10 und dem Bauchabschnitt 8 in einem Überlappungsbereich verbunden, und verbindet daher den Rückenabschnitt 10 mit dem Bauchabschnitt 8 in Artikellängsrichtung.

Die Orientierungsmarke 40, das Kennzeichnungsmittel 46 und das weitere Kennzeichnungsmittel 72 sind im vorliegenden Fall an der Rückenabschnittskomponente 11 aufgebracht also im Rückenabschnitt 10 angeordnet. Das weitere Kennzeichnungsmittel 72 vermittelt im vorliegenden Fall die Information, wo die Rückseite des Inkontinenzartikels 2 ist.

Das Kennzeichnungsmittel 46 ist auf der körperzugewandten Oberseite der Rückenabschnittskomponente 11 vorgesehen. Auf der körperabgewandten Oberseite der Rückenabschnittskomponente 11 sind das weitere Kennzeichnungsmittel 72 und die Orientierungsmarke 40 vorgesehen. Das weitere Kennzeichnungsmittel 72 und die Orientierungsmarke 40 sind hier in Form eines Farbaufdrucks vorgenommen. Durch die Aufbringung auf der körperabgewandten Oberseite der Rückenabschnittskomponente 11 wird erreicht, dass kein Abrieb des Farbaufdrucks erfolgen kann sowie ein direkter Hautkontakt mit dem Farbaufdruck vermieden wird.

## Patentansprüche

1. Verfahren zum Herstellen von Inkontinenzartikeln (2) für die Aufnahme von Körperausscheidungen mit einer Artikellängsrichtung (4) und mit einer Artikelquerrichtung (6) in einer Herstellungsmaschine, mit einem vorderen Bauchabschnitt (8) und einem hinteren Rückenabschnitt (10), die in der Artikellängsrichtung (4) zunächst voneinander beabstandet sind und dann zur Bildung eines in Quer- oder Hüftumfangsrichtung (32) durchgehenden Bauch- und Rückenbands mit einer in Hüftumfangsrichtung (22) geschlossenen Hüftöffnung (26) aufeinandergefaltet und an beidseitigen Seitennahtbereichen (24) herstellerseitig miteinander verbunden werden, und mit einem einen Absorptionskörper (14) aufweisenden Schrittabschnitt (16), der sich in der Artikellängsrichtung (4) zwischen Bauchabschnitt (8) und Rückenabschnitt (10) erstreckt, wobei der Schrittabschnitt (16), der Bauchabschnitt (8) und der Rückenabschnitt (10) gemeinsam jeweilige Beinöffnungen (28) des Inkontinenzartikels begrenzen,
wobei eine hintere Rückenabschnittskomponenten (11) bildende erste Flachmaterialbahn (34) und eine vordere Bauchabschnittskomponenten (9) bildende zweite Flachmaterialbahn (36) in einer Maschinenlängsrichtung (42), welche die spätere Artikelquerrichtung (6) bildet, endlos zugeführt werden und wobei in einer Positionierstation (60) für die Schrittabschnitte (16) die jeweiligen Schrittabschnitte (16) mit der späteren Artikellängsrichtung (4) quer zu der Maschinenlängsrichtung (42) angeordnet werden und mit der ersten und mit der zweiten Flachmaterialbahn (34, 36) für den bestimmungsgemäßen Gebrauch des Artikels verbunden werden, und
wobei an dem Bauchabschnitt (8) und/oder an dem Rückenabschnitt (10) ein Kennzeichnungsmittel (46) angebracht wird,
wobei die erste Flachmaterialbahn (34) und/oder die zweite Flachmaterialbahn (36) in Maschinenlängsrichtung (42) aufeinanderfolgende Orientierungsmarken (40) aufweist,
**dadurch gekennzeichnet,**
**dass** in der Herstellungsmaschine eine jeweilige Orientierungsmarke (40) mittels eines ersten Sensors (52) erfasst wird und dass in Abhängigkeit vom Zeitpunkt einer jeweiligen Erfassung eine in der Maschinenlängsrichtung (42) nachfolgende Druckvorrichtung (56) zum Aufbringen des Kennzeichnungsmittels (46) angesteuert wird, so dass das Kennzeichnungsmittel (46) in der Maschinenlängsrichtung (42) in vorbestimmter Relation zu der jeweiligen mittels des ersten Sensors (52) erfassten Orientierungsmarke (40) aufgebracht wird, und
**dass** in der Herstellungsmaschine eine jeweilige Orientierungsmarke (40) mittels eines zweiten Sensors (64) erfasst wird und dass in Abhängigkeit vom Zeitpunkt einer jeweiligen Erfassung die erste und/oder die zweite Flachmaterialbahn (34, 36) beschleunigt oder verzögert oder mit gleichbleibender Geschwindigkeit weitertransportiert wird, derart dass die Anordnung der jeweiligen Schrittabschnitte (16) in der Positionierstation (60) in der Maschinenlängsrichtung (42) in vorbestimmter Relation zu der jeweiligen mittels des zweiten Sensors (64) erfassten Orientierungsmarke (40) ausgeführt wird, und wobei mittels des ersten Sensors und mittels des zweiten Sensors dieselben Orientierungsmarken erfasst werden.

2. Verfahren nach Anspruch 1, **dadurch gekennzeichnet, dass** eine jeweilige Erfassung von Orientierungsmarken (40) mittels des zweiten Sensors (64) zeitlich nach einer jeweiligen Erfassung von Orientierungsmarken (40) mittels des ersten Sensors (52) ausgeführt wird.

3. Verfahren nach Anspruch 2, **dadurch gekennzeichnet, dass** der zweite Sensor (64) in der Maschinenlängsrichtung (42) der Druckvorrichtung (56) nachfolgend angeordnet ist.

4. Verfahren nach Anspruch 1, 2 oder 3, **dadurch gekennzeichnet, dass** der erste Sensor (52) und/oder der zweite Sensor (64) ein optischer oder optoelektronischer Sensor ist.

5. Verfahren nach einem oder mehreren der vorstehenden Ansprüche, **dadurch gekennzeichnet, dass** der erste Sensor (52) mit einer elektronischen Steuervorrichtung (54) für die Druckvorrichtung (56) zusammenwirkt, wobei ein weiterer Parameter von einer Transportgeschwindigkeit der Flachmaterialbahn (34, 36) in der Maschinenlängsrichtung (42) gebildet wird.

6. Verfahren nach Anspruch 5, **dadurch gekennzeichnet, dass** die Erfassung der jeweiligen Orientierungsmarke (40) mittels des ersten Sensors (52) einen Startzeitpunkt für die elektronische Steuervorrichtung (54) bildet und dass unter Verwendung dieses Startzeitpunkts ein Ereigniszeitpunkt für die Auslösung des Druckvorgangs durch die Druckvorrichtung (56) ermittelt und der Druckvorgang zu diesem Ereigniszeitpunkt ausgelöst wird.

7. Verfahren nach einem oder mehreren der vorstehenden Ansprüche, **dadurch gekennzeichnet, dass** durch Erfassen der jeweiligen Orientierungsmarke (40) mittels des zweiten Sensors (64) und der anschließenden Geschwindigkeitsänderung der Flachmaterialbahn (34, 36) eine Veränderung von Orientierungsmarkenabständen ausgeglichen wird.

8. Verfahren nach einem oder mehreren der vorstehenden Ansprüche, **dadurch gekennzeichnet, dass** als Kennzeichnungsmittel (46) eine chargenindividualisierende Information, insbesondere eine Lotnummer, aufgebracht wird.

9. Verfahren nach einem oder mehreren der vorstehenden Ansprüche, **dadurch gekennzeichnet, dass** die erste und/oder die zweite Flachmaterialbahn (34, 36) außer den Orientierungsmarken (40) je herzustellendem Inkontinenzartikel ein weiteres, insbesondere visuell wahrnehmbares Kennzeichnungsmittel (72) aufweist, welches in vorbestimmter räumlicher Anordnung zu der Orientierungsmarke (40) angeordnet ist.

10. Verfahren nach einem oder mehreren der vorstehenden Ansprüche, **dadurch gekennzeichnet, dass** die Herstellungsmaschine mit einer Geschwindigkeit der ersten und der zweiten Flachmaterialbahn (34, 36) von 100-800 m/min, insbesondere 200-600 m/min betrieben wird.

11. Verfahren nach einem oder mehreren der vorstehenden Ansprüche, **dadurch gekennzeichnet, dass** im laufenden Betrieb in einer Spleißstation (90) der Herstellungsmaschine eine jeweilige die erste und/oder die zweite Flachmaterialbahn (34, 36) durch Abrollen zuführende Flachmaterialrolle (30, 32) gegen eine neue Flachmaterialrolle (30') ausgetauscht werden kann, wobei die Flachmaterialbahn (34) der zunehmend aufgebrauchten Flachmaterialrolle (30) mit der Flachmaterialbahn (34') der neuen Flachmaterialrolle (30') prozesstauglich verbunden wird, und dass hierfür mittels eines weiteren Sensors (102) eine Orientierungsmarke (40) bei der Flachmaterialbahn (34) der zunehmend aufgebrauchten Flachmaterialrolle (30) erfasst wird.

12. Verfahren nach Anspruch 11, **dadurch gekennzeichnet dass** ein Ende der Flachmaterialbahn (34) der zunehmend aufgebrauchten Flachmaterialrolle (30) in vorbestimmter Verzögerung zum Stillstand gebracht wird, so dass sich die Orientierungsmarke (40) in einem vorbestimmten Abstand (d) in der Maschinenlängsrichtung (42) zu einer nachfolgenden Orientierungsmarke (40) der vorpositionierten Flachmaterialbahn (34') der neuen Flachmaterialrolle (30') befindet und die Flachmaterialbahnen (34, 34') dann prozesstauglich verbunden werden, so dass die Orientierungsmarken (40) der entstehenden Flachmaterialbahn denselben vorbestimmten Abstand (104) zueinander haben wie die Orientierungsmarken (40) der Flachmaterialbahn (34) der zunehmend aufgebrauchten Flachmaterialrolle (30).

13. Verfahren nach Anspruch 11, **dadurch gekennzeichnet dass** eine Bahngeschwindigkeit der zunehmend aufgebrauchten Flachmaterialbahn und eine Bahngeschwindigkeit der neuen Flachmaterialbahn aneinander angeglichen werden, so dass die zunehmend aufgebrauchte Flachmaterialbahn und die neue Flachmaterialbahn zum Zeitpunkt des Verbindens mit weitestgehend gleicher Geschwindigkeit in der Herstellungsmaschine geführt werden, und die Flachmaterialbahnen (34, 34') dann prozesstauglich verbunden werden, so dass die Orientierungsmarken (40) der entstehenden Flachmaterialbahn denselben vorbestimmten Abstand (104) zueinander haben wie die Orientierungsmarken (40) der Flachmaterialbahn (34) der zunehmend aufgebrauchten Flachmaterialrolle (30).

14. Verfahren nach einem oder mehreren der vorstehenden Ansprüche 11-13, **dadurch gekennzeichnet, dass** mittels des weiteren Sensors (102) dieselben Orientierungsmarken (40) erfasst werden wie mit dem ersten und/oder zweiten Sensor (52, 64).

15. Verfahren nach einem oder mehreren der vorstehenden Ansprüche, **dadurch gekennzeichnet, dass** die Flachmaterialbahnen (34, 36) von elastisch dehnbaren oder undehnbaren Vliesmaterialien oder Vlies/Film-Materialien gebildet sind.

16. Verfahren nach einem oder mehreren der vorstehenden Ansprüche, **dadurch gekennzeichnet, dass** der Spleißstation (90) in der Maschinenlängsrichtung (42) nachfolgend und dem ersten Sensor (52) und der Druckstation (48) vorgeordnet eine Bahnspeichereinrichtung (100) vorgesehen und betrieben wird.

17. Verfahren nach einem oder mehreren der vorstehenden Ansprüche, **dadurch gekennzeichnet, dass** der Druckstation (48) in der Maschinenlängsrichtung (42) nachgeordnet und dem zweiten Sensor (64) und der Ablege- und Fügevorrichtung (70) vorgeordnet eine Bahnantriebseinrichtung (68) vorgesehen und betrieben wird.

## Claims

1. A method for the production of incontinence articles (2) for the receptacle of body excretions with an article longitudinal direction (4) and with an article transverse direction (6) in a manufacturing machine, with a front abdominal portion (8) and a rear back portion (10) which are initially spaced apart from one another in the longitudinal direction (4) of the article and are then folded onto one another to form a belly and back band which is continuous in the transverse or hip circumferential direction (32) and has a hip opening (26) which is closed in the hip circumferential direction (22), and are connected to one another at side seam areas (24) on both sides on the manufacturer's side, and
with a crotch portion (16) having an absorbent body (14) which extends in the longitudinal direction (4) of the article between the abdominal portion (8) and the back portion (10), wherein the crotch portion (16), the abdominal portion (8) and the back portion (10) together delimit respective leg openings (28) of the incontinence article,
wherein a first flat material web (34) forming rear back portion components (11) and a second flat material web (36) forming front belly portion components (9) are fed endlessly in a longitudinal machine direction (42), which forms the later transverse article direction (6), and wherein in a positioning station (60) for the crotch portions (16) the respective crotch portions (16) are arranged with the later longitudinal direction (4) of the article transverse to the longitudinal direction (42) of the machine and are connected to the first and to the second flat material web (34, 36) for the intended use of the article, and
wherein a marking means (46) is attached to the belly portion (8) and/or to the back portion (10),
wherein the first flat material web (34) and/or the second flat material web (36) has successive orientation marks (40) in the longitudinal direction (42) of the machine,
**characterized in that**
a respective orientation mark (40) is detected in the manufacturing machine by means of a first sensor (52) and depending on the time of a respective detection, a subsequent printing device (56) in the longitudinal direction (42) of the machine is actuated for applying the marking means (46), so that the marking means (46) is applied in the longitudinal direction (42) of the machine in a predetermined relationship to the respective orientation mark (40) detected by means of the first sensor (52), and
a respective orientation mark (40) is detected in the manufacturing machine by means of a second sensor (64) and depending on the time of a respective detection, the first and/or the second flat material web (34, 36) is accelerated or decelerated or transported further at a constant speed, in such a manner that the arrangement of the respective crotch portions (16) in the positioning station (60) is carried out in the longitudinal direction (42) of the machine in predetermined relation to the respective orientation mark (40) detected by means of the second sensor (64), and wherein the same orientation marks are detected by means of the first sensor and by means of the second sensor.

2. The method according to claim 1, **characterized in that** a respective detection of orientation marks (40) by means of the second sensor (64) is carried out at a time after a respective detection of orientation marks (40) by means of the first sensor (52).

3. The method according to claim 2, **characterized in that** the second sensor (64) is arranged downstream in the longitudinal machine direction (42) of the printing device (56).

4. The method according to claim 1, 2 or 3, **characterized in that** the first sensor (52) and/or the second sensor (64) is an optical or optoelectronic sensor.

5. The method according to any of the preceding claims, **characterized in that** the first sensor (52) cooperates with an electronic control device (54) for the printing device (56), wherein a further parameter is formed by a transport speed of the flat material web (34, 36) in the longitudinal machine direction (42).

6. The method according to claim 5, **characterized in that** the detection of the respective orientation mark (40) by means of the first sensor system (52) forms a start time for the electronic control device (54) and **in that**, using this start time, an event time for triggering the printing process by the printing device (56) is determined and the printing process is triggered at this event time.

7. The method according to any of the preceding claims, **characterized in that** a change in orientation mark spacing is compensated for by detecting the respective orientation mark (40) by means of the second sensor (64) and subsequently changing the speed of the flat material web (34, 36).

8. The method according to any of the preceding claims, **characterized in that** a batch-individualizing information, particularly a lot number, is applied as marking means (46).

9. The method according to any of the preceding claims, **characterized in that** the first and/or the second flat material web (34, 36) has, in addition to the orientation marks (40), a further, particularly visually perceptible marking means (72) for each incontinence article to be produced, which marking means is arranged in a predetermined spatial arrangement relative to the orientation mark (40).

10. The method according to any of the preceding claims, **characterized in that** the manufacturing machine is operated at a speed of the first and second flat material webs (34, 36) of 100-800 m/min, particularly 200-600 m/min.

11. The method according to any of the preceding claims, **characterized in that**, during operation in a splicing station (90) of the manufacturing machine, a respective flat material roll (30, 32) feeding the first and/or the second flat material web (34, 36) by unwinding can be exchanged for a new flat material roll (30'), wherein the flat material web (34) of the increasingly used-up flat material roll (30) is connected to the flat material web (34') of the new flat material roll (30') in a process-compatible manner, and **in that** an orientation mark (40) on the flat material web (34) of the increasingly used-up flat material roll (30) is detected for this purpose by means of a further sensor (102) .

12. The method according to claim 11, **characterized in that** one end of the flat material web (34) of the increasingly used-up flat material roll (30) is brought to a standstill with a predetermined delay, so that the orientation mark (40) is located at a predetermined distance (d) in the longitudinal direction (42) of the machine from a subsequent orientation mark (40) of the pre-positioned flat material web (34') of the new flat material roll (30'), and the flat material webs (34, 34') are then connected in a manner suitable for the process, so that the orientation marks (40) of the resulting flat material web have the same predetermined distance (104) from one another as the orientation marks (40) of the flat material web (34) of the increasingly used-up flat material roll (30).

13. The method according to claim 11, **characterized in that** a web speed of the increasingly used-up flat material web and a web speed of the new flat material web are matched to one another, so that the increasingly used-up flat material web and the new flat material web are guided at substantially the same speed in the manufacturing machine at the time of connection, and the flat material webs (34, 34') are then connected in a process-compatible manner, so that the orientation marks (40) of the resulting flat material web have the same predetermined distance (104) from one another as the orientation marks (40) of the flat material web (34) of the increasingly used-up flat material roll (30) .

14. The method according to any of the preceding claims 11-13, **characterized in that** by means of the further sensor (102) the same orientation marks (40) are detected as with the first and/or second sensor (52, 64) .

15. The method according to any of the preceding claims, **characterized in that** the flat material webs (34, 36) are formed by elastically stretchable or non-stretchable nonwoven materials or nonwoven/film materials.

16. The method according to any of the preceding claims, **characterized in that** a web storage device (100) is provided and operated downstream of the splicing station (90) in the longitudinal direction (42) of the machine and upstream of the first sensor (52) and the printing station (48).

17. The method according to any of the preceding claims, **characterized in that** a web drive device (68) is provided and operated downstream of the printing station (48) in the longitudinal direction (42) of the machine and upstream of the second sensor (64) and the depositing and joining device (70).

## Revendications

1. Procédé de fabrication d'articles d'incontinence (2) destinés à recueillir les excrétions corporelles et ayant une direction longitudinale d'article (4) et une direction transversale d'article (6), dans une machine de fabrication, comprenant une portion ventrale avant (8) et une portion dorsale arrière (10) qui sont d'abord espacées l'une de l'autre dans la direction longitudinale d'article (4) et qui, ensuite, pour la formation d'une bande ventrale et dorsale continue dans le sens transversal ou dans le sens du tour de hanches (32), avec une ouverture de hanche (26) fermée dans le sens du tour de hanches (22), sont pliées l'une sur l'autre et reliées entre elles par le fabricant au niveau de zones de couture latérale (24) situées de part et d'autre, et
comprenant une portion d'entrejambe (16) qui présente un corps absorbant (14) et s'étend dans la direction longitudinale d'article (4) entre la portion ventrale (8) et la portion dorsale (10), dans lequel la portion d'entrejambe (16), la portion ventrale (8) et la portion dorsale (10) délimitent ensemble des ouvertures de jambe (28) respectives de l'article d'incontinence, dans lequel une première bande de matériau plat (34) formant des composants arrière de portion dorsale (11) et une deuxième bande de matériau plat (36) formant des composants avant de portion ventrale (9) sont amenées sans fin dans une direction longitudinale de machine (42) qui forme la direction transversale d'article (6) ultérieure, et dans lequel, dans un poste de positionnement (60) pour les portions d'entrejambe (16), les portions d'entrejambe (16) respectives sont disposées de manière à ce que la direction longitudinale d'article ultérieure (4) soit transversale à la direction longitudinale de machine (42) et sont reliées aux première et deuxième bandes de matériau plat (34, 36) pour l'utilisation prévue de l'article, et
dans lequel un moyen de marquage (46) est fixé à la portion ventrale (8) et/ou à la portion dorsale (10),
dans lequel la première bande de matériau plat (34) et/ou la deuxième bande de matériau plat (36) présente(nt) des marques d'orientation (40) successives dans la direction longitudinale de machine (42),
**caractérisé par le fait**
**que** dans la machine de fabrication, une marque d'orientation (40) respective est détectée au moyen d'un premier capteur (52) et que, en fonction du moment d'une détection respective, un dispositif d'impression (56) situé en aval dans la direction longitudinale de machine (42) est commandé pour appliquer le moyen de marquage (46) de sorte que le moyen de marquage (46) soit appliqué dans la direction longitudinale de machine (42) dans une relation prédéterminée par rapport à la marque d'orientation (40) respective détectée au moyen du premier capteur (52), et
**que**, dans la machine de fabrication, une marque d'orientation (40) respective est détectée au moyen d'un deuxième capteur (64) et que, en fonction du moment d'une détection respective, la première et/ou la deuxième bande de matériau plat (34, 36) est accélérée ou retardée ou transporté davantage à une vitesse constante de telle sorte que la disposition des portions d'entrejambe (16) respectives dans le poste de positionnement (60) dans la direction longitudinale de machine (42) s'effectue dans une relation prédéterminée par rapport à la marque d'orientation (40) respective détectée au moyen du deuxième capteur (64), et dans lequel les mêmes marques d'orientation sont détectées au moyen du premier capteur et au moyen du deuxième capteur.

2. Procédé selon la revendication 1, **caractérisé par le fait qu'**une détection respective de marques d'orientation (40) au moyen du deuxième capteur (64) est effectuée temporellement après une détection respective de marques d'orientation (40) au moyen du premier capteur (52).

3. Procédé selon la revendication 2, **caractérisé par le fait que** le deuxième capteur (64) est disposé en aval du dispositif d'impression (56) dans la direction longitudinale de machine (42).

4. Procédé selon la revendication 1, 2 ou 3, **caractérisé par le fait que** le premier capteur (52) et/ou le deuxième capteur (64) est un capteur optique ou optoélectronique.

5. Procédé selon une ou plusieurs des revendications précédentes, **caractérisé par le fait que** le premier capteur (52) agit de concert avec un dispositif de commande électronique (54) pour le dispositif d'impression (56), dans lequel un autre paramètre est formé par une vitesse de transport de la bande de matériau plat (34, 36) dans la direction longitudinale de machine (42).

6. Procédé selon la revendication 5, **caractérisé par le fait que** la détection de la marque d'orientation (40) respective au moyen du premier capteur (52) forme un moment de démarrage pour le dispositif de commande électronique (54) et qu'un moment d'événement pour le déclenchement de l'opération d'impression par le dispositif d'impression (56) est détecté en utilisant ce moment de démarrage et que l'opération impression (56) est déclenché à ce moment d'événement.

7. Procédé selon une ou plusieurs des revendications précédentes, **caractérisé par le fait qu'**un changement de distances de marques d'orientation est compensé par la détection de la marque d'orientation (40) respective au moyen du deuxième capteur (64) et le changement de vitesse de la bande de matériau plat (34, 36), qui a lieu ensuite.

8. Procédé selon une ou plusieurs des revendications précédentes, **caractérisé par le fait qu'**une information individualisant un lot, en particulier un numéro de lot, est appliquée en tant que moyen de marquage (46).

9. Procédé selon une ou plusieurs des revendications précédentes, **caractérisé par le fait que** la première et/ou la deuxième bande de matériau plat (34, 36) présente, outre les marques d'orientation (40), un autre moyen de marquage (72), en particulier visuellement perceptible, pour chaque article d'incontinence à fabriquer, qui est disposé dans une disposition spatiale prédéterminée par rapport à la marque d'orientation (40).

10. Procédé selon une ou plusieurs des revendications précédentes, **caractérisé par le fait que** la machine de fabrication fonctionne à une vitesse des première et deuxième bandes de matériau plat (34, 36) comprise entre 100 et 800 m/min, en particulier entre 200 et 600 m/min.

11. Procédé selon une ou plusieurs des revendications précédentes, **caractérisé par le fait que**, pendant le fonctionnement en cours, dans un poste de jonction (90) de la machine de fabrication, un rouleau de matériau plat (30, 32) respectif qui amène par déroulement la première et/ou la deuxième bande de matériau plat (34, 36) peut être remplacée par un nouveau rouleau de matériau plat (30'), dans lequel la bande de matériau plat (34) du rouleau de matériau plat (30) qui est de plus en plus consommé est reliée à la bande de matériau plat (34') du nouveau rouleau de matériau plat (30') d'une manière adaptée au processus, et que, à cet effet, une marque d'orientation (40) est détectée au moyen d'un autre capteur (102) sur la bande de matériau plat (34) du rouleau de matériau plat (30) de plus en plus consommé.

12. Procédé selon la revendication 11, **caractérisé par le fait qu'**une extrémité de la bande de matériau plat (34) du rouleau de matériau plat (30) de plus en plus consommé est arrêtée dans un retard prédéterminé de sorte que la marque d'orientation (40) se trouve à une distance prédéterminée (d) dans la direction longitudinale de machine (42) par rapport à une marque d'orientation (40) suivante de la bande de matériau plat (34') prépositionnée du nouveau rouleau de matériau plat (30') et que les bandes de matériau plat (34, 34') sont ensuite reliées d'une manière adaptée au processus de sorte que les marques d'orientation (40) de la bande de matériau plat résultante présentent la même distance (104) prédéterminée les unes des autres que les marques d'orientation (40) de la bande de matériau plat (34) du rouleau de matériau plat (30) qui est de plus en plus consommé.

13. Procédé selon la revendication 11, **caractérisé par le fait qu'**une vitesse de bande de la bande de matériau plat de plus en plus consommée et une vitesse de bande de la nouvelle bande de matériau plat sont ajustées l'une à l'autre de sorte que la bande de matériau plat de plus en plus consommée et la nouvelle bande de matériau plat sont guidées dans la machine de fabrication à peu près à la même vitesse au moment de la jonction, et que les bandes de matériau plat (34, 34') sont ensuite reliées d'une manière adaptée au processus de sorte que les marques d'orientation (40) de la bande de matériau plat résultante présentent la même distance prédéterminée (104) les unes des autres que les marques d'orientation (40) de la bande de matériau plat (34) du rouleau de matériau plat (30) de plus en plus consommé.

14. Procédé selon une ou plusieurs des revendications précédentes 11 à 13, **caractérisé par le fait qu'**au moyen de l'autre capteur (102) sont détectées les mêmes marques d'orientation (40) qu'au moyen du premier et/ou du deuxième capteur (52, 64).

15. Procédé selon une ou plusieurs des revendications précédentes, **caractérisé par le fait que** les bandes de matériau plat (34, 36) sont formées par des matériaux non tissés ou des matériaux non tissés/film élastiquement étirables ou inextensibles.

16. Procédé selon une ou plusieurs des revendications précédentes, **caractérisé par le fait qu'**un dispositif de stockage de bande (100) est prévu et actionné en aval du poste de jonction (90) dans la direction longitudinale de machine (42) et en amont du premier capteur (52) et du poste d'impression (48).

17. Procédé selon une ou plusieurs des revendications précédentes, **caractérisé par le fait qu'**un dispositif d'entraînement de bande (68) est prévu et actionné en aval du poste d'impression (48) dans la direction longitudinale de machine (42) et en amont du deuxième capteur (64) et du dispositif de dépôt et de jonction (70) .
